(19) Europäisches Patentamt
European Patent Office
Office européen des brevets

(11) Veröffentlichungsnummer : **0 017 207**
**B1**

(12) # EUROPÄISCHE PATENTSCHRIFT

(45) Veröffentlichungstag der Patentschrift : **09.03.83**

(51) Int. Cl.³ : **A 61 K 7/46**, C 11 D 1/722

(21) Anmeldenummer : **80101703.9**

(22) Anmeldetag : **31.03.80**

(54) **Stabile wässrige oder wässrig-alkoholische Lösungen von fettlöslichen Parfümölen.**

(30) Priorität : **04.04.79 DE 2913467**

(43) Veröffentlichungstag der Anmeldung : **15.10.80 Patentblatt 80/21**

(45) Bekanntmachung des Hinweises auf die Patenterteilung : **09.03.83 Patentblatt 83/10**

(84) Benannte Vertragsstaaten : **AT BE CH DE FR GB IT NL SE**

(56) Entgegenhaltungen :
**DE A 2 062 463**
**DE A 2 252 186**
**DE A 2 536 597**
**DE A 2 639 293**
**DE A 2 644 542**
**DE B 1 132 730**
**DE C 865 510**
**FR A 1 159 324**
**FR A 2 397 188**

**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf 1 (DE)**

(72) Erfinder : **Meffert, Alfred, Dr.**
**Itterstrasse 33**
**D-4000 Düsseldorf 13 (DE)**
Erfinder : **Scheuermann, Fanny, Dr., geb. Esano-Solomon**
**Volmerswerther Strasse 66**
**D-4000 Düsseldorf (DE)**
Erfinder : **Werdehausen, Achim, Dr.**
**Röntgenstrasse 37**
**D-5657 Haan (DE)**

**EP 0 017 207 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Stabile wäßrige oder wäßrig-alkoholische Lösungen von fettlöslichen Parfümölen

Die Erfindung betrifft stabile, wäßrige oder wäßrig-alkoholische Lösungen von fettlöslichen Parfümölen, die als Lösungsvermittler propoxylierte und anschließend ethoxylierte Hydroxyalkylether enthalten.

Zur Parfümierung klarer, wäßriger oder niedrigprozentiger alkoholischer Kosmetika wie Gesichts-, Rasier- und Haarwässer können in vielen Fällen wasserlösliche Parfümöle verwendet werden. Der Großteil der ätherischen Riechstoffe, Parfümöle und Aromen sind jedoch öllösliche Produkte, die erst durch den Zusatz sogenannter Lösungsvermittler in klare, stabile, wäßrige oder wäßrigalkoholische Lösungen gebracht werden können. Es ist bereits bekannt, für die Solubilisierung öllöslicher Produkte verschiedene Lösungsvermittler, wie z. B. Monofettsäureester von Polyolen wie Sorbitanmonostearat oder verschiedene Ethylenoxidanlagerungsverbindungen wie polyoxethyliertes Rizinusöl einzusetzen.

Aus der DE-A-25 36 597 sind z. B. Wasser-in-Öl-Emulgatoren bekannt, die durch Umsetzung von Fettalkoholen mit Epichlorhydrin, Reaktion der erhaltenen Glycidylether mit mehrwertigen Alkoholen und anschließende Oxalkylierung erhalten werden. Für diese Stoffe wurde auch die Verwendung als Lösungsvermittler für ätherische Öle beschrieben. Aus der DE-A-26 39 293 sind klare wäßrige oder wäßrig-alkoholische Lösungen fettlöslicher Parfümöle bekannt, die als Lösungsvermittler Hydroxyalkylester- und/oder N-(Hydroxyalkyl) amid-Oxethylate enthalten.

Ein wesentlicher Nachteil der bisher verwendeten Lösungsvermittler besteht darin, daß relativ große Zusatzmengen erforderlich sind, um die gewünschten und erforderlichen Mengen an Parfümölen in eine stabile, wäßrige oder niedrigprozentige alkoholische Lösung zu bringen. Ein weiterer Nachteil besteht darin, daß ihre lösungsvermittelnde Wirkung meist sehr spezifisch ist und sich auf eine eingeschränkte Zahl von Parfümölen erstreckt.

Diese Aufgabe, Lösungsvermittler aufzufinden, die bereits bei Zusatz geringer Mengen eine möglichst große Zahl verschiedener Parfümöle in der gewünschten und erforderlichen Konzentration in eine klare, stabile, wäßrige oder niedrigprozentige alkoholische Lösung zu bringen vermögen, war durch den Einsatz von Hydroxy-alkyletheroxethylaten, wie er in der deutschen Offenlegungsschrift 27 31 218 beschrieben ist, weitestgehend gelöst worden. Wenn auch die dort genannten Hydroxyalkyletheroxethylate hervorragende Solubilisierungseigenschaften für Parfümöle aufweisen, so lassen sie aufgrund ihrer Konsistenz und teilweisen Inhomogenität in verarbeitungstechnischer Hinsicht manche Wünsche offen und zwar müssen die Produkte vor ihrem Einsatz aufgeschmolzen oder homogenisiert werden.

Es bestand daher die Aufgabe Lösungsvermittler zu entwickeln, die alle positiven anwendungstechnischen Eigenschaften der Hydroxyalkyletheroxethylate besitzen und darüber hinaus klarflüssige, leicht verarbeitbare Produkte darstellen, so daß mit ihrer Hilfe die Herstellung der erfindungsgemäßen stabilen wäßrigen oder wäßrig-alkoholischen Lösungen fettlöslicher Parfümöle ohne Schwierigkeiten möglich ist.

Diese Aufgabe wird erfindungsgemäß gelöst durch klare, stabile, wäßrige oder wäßrig-alkoholische Lösungen fettlöslicher Parfümöle, gekennzeichnet durch einen Gehalt an Produkten, die dadurch erhalten werden, daß man Epoxyalkane der Formel

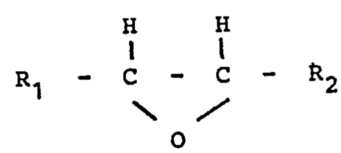

in der $R_1$ und $R_2$ Wasserstoff oder Alkylreste, mit der Maßgabe, daß die Summe der Kohlenstoffatome von $R_1 + R_2$ im Bereich von 6 bis 20 liegt, mit polyfunktionellen Alkoholen, die bis zu 10 Kohlenstoffatome und bis zu 4 alkoholische Hydroxylgruppen enthalten, in einem Molverhältnis Epoxyalkan : Alkohol von 1 : 1,1 bis 1 : 10, vorzugsweise 1 : 2 bis 1 : 6 bei 50 bis 130 °C in Abwesenheit von Lösungsmitteln und in Gegenwart von 0,05 bis 10 g Schwefelsäure oder aromatischen Sulfonsäuren mit höchstens 8 Kohlenstoffatomen pro Mol umzusetzendes Epoxyalkan umsetzt, das erhaltene Reaktionsprodukt nach Neutralisation der Säure und Abdestillieren des nicht umgesetzten Alkohols zunächst pro Mol Etheralkohol mit 0,5 bis 4 Mol Propylenoxid und anschließend mit 7 bis 14 Mol Ethylenoxid umsetzt.

Epoxyalkane werden aus den entsprechenden Olefinen bzw. Olefingemischen durch Epoxidation nach bekannten Verfahren erhalten. Zu den 1,2-Epoxyalkanen kommt man über 1,2-Monoolefine, die beispielsweise durch Polymerisation von Ethylen mit organischen Aluminiumverbindungen als Katalysatoren oder durch thermisches Cracken von Paraffinkohlenwasserstoffen erhalten werden. Beispiele für bevorzugt verwendbare Epoxyalkane sind die Verbindungen 1,2-Epoxydodecan, 1,2-Epoxytetradecan, 1,2-Epoxyhexadecan, 1,2-Epoxyoctadecan und Epoxidgemische wie z. B. $C_{12/14}$-1,2-Epoxid mit ca. 70 Gew.-% $C_{12}$- und ca. 30 Gew.-% $C_{14}$-Epoxyalkan oder $C_{16/18}$-1,2-Epoxid mit ca. 40 Gew.-% $C_{16}$- und ca. 60 Gew.-% $C_{18}$-Epoxyalkan.

Die innenständigen Epoxyalkane erhält man beispielsweise durch Epoxydierung von Olefinen oder Olefingemischen, die durch katalytische Dehydrierung oder durch Chlorierung/Dehydrierung von linearen Paraffinkohlenwasserstoffen hergestellt werden. Monoolefine mit innenständiger

Doppelbindung können auch durch Isomerisierung von α-Olefinen hergestellt werden. Bevorzugt eingesetzte innenständige Epoxyalkane (i-Epoxyalkane) aus einer $C_{11/14}$-Olefinfraktion enthielten ca. 22 Gew.-% $C_{11}$-, ca. 30 Gew.-% $C_{12}$-, ca. 26 Gew.-% $C_{13}$-, ca. 22 Gew.-% $C_{14}$-Epoxyalkan. Ein ebenfalls geeignetes Gemisch innenständiger Epoxyalkane einer $C_{15/18}$-Olefin-Fraktion enthielt ca. 26 Gew.-% $C_{15}$-, ca. 35 Gew.-% $C_{16}$-, ca. 32 Gew.-% $C_{17}$- und ca. 7 Gew.-% $C_{18}$-Epoxyalkan.

Geeignete polyfunktionelle Alkohole, die bis zu 10 Kohlenstoffatome und bis zu 4 alkoholische OH-Gruppen enthalten, sind beispielsweise Ethandiol-1,2, Propandiol-1,2, Glycerin und 1,1,1-Tris-(hydroxymethyl)-propan (Trimethylolpropan). Besondere Bedeutung ist dabei dem Ethandiol-1,2 beizumessen.

Ist das Angebot an polyfunktionellen Alkoholen im Reaktionsansatz hoch, enthält das Reaktionsprodukt einen hohen Anteil an Umsetzungsprodukten aus einem Mol Epoxyalkan mit einem Mol Alkohol. Bei geringem Alkohol-Angebot entstehen in zunehmenden Maße Sekundär-Umsetzungsprodukte aus einem Mol Epoxyalkan mit einem Mol bereits umgesetztem Etheralkohol. Als besonders vorteilhaft hat sich ein Molverhältnis von 2 bis 6 Mol Alkohol pro Mol Epoxyalkan erwiesen ; hierbei beträgt der Anteil an Primärreaktionsprodukten im Reaktionsgemisch 70 bis über 99 Mol-%.

Die Menge der als Katalysator einzusetzenden starken Säuren hängt in einem gewissen Maße von der Art des Epoxyalkans und des Alkohols ab. Sie liegt bei 0,05 bis 10 g Säure pro Mol des umzusetzenden Epoxyalkans ; hiermit wird eine hohe Umsetzungsgeschwindigkeit erzielt.

Als katalytisch wirksame starke Säure, die bei hoher Aktivität unter den Bedingungen des Verfahrens zu hellfarbigen bis farblosen Produkten führt, ohne nennenswerte Korrosion an den Anlageteilen zu verursachen, hat sich konzentrierte Schwefelsäure erwiesen, mit der man eine vollständige Umsetzung innerhalb von 1 bis 5 Stunden durchführen kann. Die Vollständigkeit der Umsetzung kann dabei durch Messung der Epoxid-Zahl oder gaschromatographisch geprüft werden. Ähnliche Ergebnisse werden beim Einsatz von aromatischen Sulfonsäuren mit höchstens 8 Kohlenstoffatomen, beispielsweise Benzolsulfonsäure, Xylolsulfonsäuren und insbesondere p-Toluolsulfonsäure erhalten.

Besonders hellfarbige Umsetzungsprodukte erhält man, wenn man die Umsetzung bei Reaktionstemperaturen zwischen 70 und 90 °C durchführt.

Nach beendeter Umsetzung wird zunächst die als Katalysator eingesetzte Säure neutralisiert. Hierzu können grundsätzlich alle starken anorganischen Basen, zum Beispiel NaOH, KOH, LiOH oder organische Basen, zum Beispiel Alkalialkoholat oder quartäre Ammoniumbasen verwendet werden. Besonders geeignet ist dabei Natriummethylat.

Überschüssigen Alkohol trennt man nach der Neutralisation der Säure durch Destillation unter vermindertem Druck ab. Hierbei ist eine Destillationstemperatur von höchstens 150 °C einzuhalten ; bei höheren Temperaturen erfolgt eine Verfärbung der Umsetzungsprodukte. Je nach der Dauer der Temperatureinwirkung auf das Reaktionsgemisch kann es zur Herstellung hellfarbiger Produkte zweckmäßig sein, durch Verringerung des Destillationsdruckes auch bei niedrigeren Destillationstemperaturen als 150 °C den Alkohol abzutrennen.

Die erhaltenen Etheralkohole werden zunächst nach bekannten Verfahren in Gegenwart von Natriumethylat als Katalysator mit den entsprechenden Mengen Propylen-oxid und anschließend mit Ethylenoxid im Autoklaven jeweils bei Reaktionstemperaturen zwischen 160 und 180 °C umgesetzt. Die erhaltenen Endprodukte stellen klare Flüssigkeiten dar, die auch bei niedrigen Temperaturen nicht austrüben.

Besondere Bedeutung als Lösungsvermittler in den erfindungsgemäßen klaren, stabilen, wäßrigen oder wäßrig-alkoholischen Lösungen fettlöslicher Parfümöle kommt den sich von endständigen $C_{12}$-$C_{14}$-, $C_{12}$-$C_{18}$-, $C_{14}$-$C_{16}$- bzw. $C_{16}$-$C_{18}$-Epoxyalkanen und den sich von innenständigen $C_{15}$-$C_{18}$-Epoxyalkanen durch Umsetzung mit Ethandiol-1,2 erhaltenen Etheralkoholen ableitenden Produkten zu.

Als bevorzugte Verbindungen sind Umsetzungsprodukte von

α-$C_{14/16}$-Epoxid + Glykol + 1 PO + 9 EO,
α-$C_{12/18}$-Epoxid + Glykol + 1,2 PO + 9 EO,
α-$C_{12/14}$-Epoxid + Glykol + 1 PO + 10 EO,
α-$C_{16/18}$-Epoxid + Glykol + 1 PO + 11 EO,
α-$C_{14/16}$-Epoxid + Glykol + 1,2 PO + 9 EO,
i-$C_{15/18}$-Epoxid + Glykol + 1 PO + 9 EO,
i-$C_{15/18}$-Epoxid + Glykol + 1,2 PO + 9 EO zu nennen.

Als fettlösliche Parfümöle kommen natürliche oder synthetische ätherische Öle aller Art in Betracht, wie zum Beispiel Orangenöl, Pineöl, Pfefferminzöl, Eucalyptusöl, Citronenöl, Nelkenblätteröl, Cerdernholzöl, Bergamottöl, Rosmarinöl, Patschouliöl, Lavandinöl, Spiköl, Rosenöl, Vetiveröl, Fenchelöl, Anisöl, Thymianöl, Geraniumöl, Lavendelöl, Menthol sowie synthetische, öllösliche Parfümöle aus der Gruppe der Aldehyde, Ester und Polyenverbindungen.

Die Mengenverhältnisse von fettlöslichem Parfümöl und Hydroxyalkylether-alkoxylat in den erfindungsgemäßen, klaren, stabilen, wäßrigen oder wäßrig-alkoholischen Lösungen können sich in weiten Grenzen bewegen und richten sich nach der Art des Parfümöls, der Art des Lösungsvermittlers, nach dem Alkoholgehalt und sonstigen Begleitstoffen, die in der Lösung zugegen sind.

Die Hydroxyalkylether-alkoxylate können in den erfindungsgemäßen Lösungen in Mengen von 0,1 bis

20 Gew.-% zugegen sein, werden sich jedoch im allgemeinen in Mengen von 0,5 bis 5 Gew.-% bewegen. Hierbei ist davon auszugehen, daß die gewünschten Mengen an Parfümöl die Grenzen von 0,1 bis 1 Gew.-%, bezogen auf die gesamte Lösung, nicht wesentlich unter- beziehungsweise überschreiten.

Die Herstellung der erfindungsgemäßen klaren, stabilen, wäßrigen oder wäßrig-alkoholischen Lösungen von fettlöslichen Parfümölen kann in an sich bekannter Weise erfolgen, indem man entsprechende Konzentrate aus Parfümöl und Hydroxyalkyletheralkoxylat im gewünschten Mengenverhältnis mit Wasser oder einem Alkohol-Wasser-Gemisch versetzt.

Die nachfolgenden Beispiele sollen den Gegenstand der Erfindung näher erläutern.

## Beispiele

Zunächst soll die Herstellung der Hydroxyalkyletheralkoxylate beschrieben werden, die in den erfindungsgemäßen wäßrigen oder wäßrig-alkoholischen Lösungen fettlöslicher Parfümöle zum Einsatz kommen.

A. Herstellung des Produktes $\alpha$-$C_{12/18}$-Epoxid + Glykol + 1,2 PO + 9 EO
(5 % $C_{12}$, 35 % $C_{14}$, 60 % $C_{16/18}$)

1 066 g eines Gemisches von 5 % $\alpha$-$C_{12}$-, 35 % $\alpha$-$C_{14}$-, 24 % $\alpha$-$C_{16}$- und 36 % $\alpha$-$C_{18}$-Epoxid (4,25 Mol) wurden zu 1 315 g Ethylenglykol (21,2 Mol) getropft, welches mit 1,1 g konzentrierter Schwefelsäure versetzt und auf 70-75 °C erwärmt worden war. Die Temperatur wurde über den Epoxidzulauf oder eventuelle Kühlung während der Reaktion zwischen 75 °C und 80 °C gehalten. Nach Umsetzung des Epoxids wurde der Katalysator mit 3,5 g Natriummethylat (30 %ig) neutralisiert und das überschüssige Glykol im Vakuum abdestilliert. Es wurden 1 525 g Etheralkohol erhalten.

416 g des Etheralkohols (1,25 Mol) wurden mit 7,9 g Natriummethylatlösung (30 %ig) als Katalysator gemischt, im Autoklaven zunächst mit 87,5 g Propylenoxid (1,5 Mol) und anschließend mit 496,5 g Ethylenoxid (11,3 Mol) bei Reaktionstemperaturen zwischen 160 °C und 180 °C umgesetzt. Es wurde dabei mit maximal 4,5 bar Überdruck gefahren. Es wurden 1 000 g Alkoxylat erhalten.

In analoger Weise wurden die nachstehend aufgeführten Hydroxyalkylether-alkoxylate, die in den Beispielen eingesetzt wurden, hergestellt.

B. $\alpha$-$C_{12/18}$-Epoxid + Glykol + 1,2 PO + 9 EO
(10 % $C_{12}$, 30 % $C_{14}$, 60 % $C_{16/18}$).

C. $\alpha$-$C_{14/18}$-Epoxid + Glykol + 1 PO + 9 EO.

D. $\alpha$-$C_{12/14}$-Epoxid + Trimethylolpropan + 1 PO + 10 EO.

E. $\alpha$-$C_{16/18}$-Epoxid + Trimethylolpropan + 1 PO + 11 EO.

F. i-$C_{11/14}$-Epoxid + Glykol + 1,2 PO + 9 EO.

G. $\alpha$-$C_{12/14}$-Epoxid + Glykol + 1 PO + 9 EO.

Zur Prüfung der lösungsvermittelnden Eigenschaften wurden folgende Versuche durchgeführt. Es wurde jeweils eine 1 %ige wäßrige Lösung des eingesetzten Parfümöls hergestellt. Zu diesem Zweck wurde zunächst das Parfümöl mit jeweiligen Lösungsvermittler im bestimmten Mengenverhältnis zusammengerührt und anschließend soviel Wasser zugegeben, daß eine 1 %ige Lösung, bezogen auf Parfümöl, entstand. Als Verhältnis Lösungsvermittler : Parfümöl wurde 7 : 3, beziehungsweise 8 : 2 gewählt, was einer etwa 2- bis 4-fachen Menge Lösungsvermittler auf Parfümöl entspricht.

Als Testsubstanzen dienten 10 ätherische Öle unterschiedlicher Zusammensetzung und Polarität.

| | |
|---|---|
| 01 Rosmarinöl | 06 Orangenöl |
| 02 Bergamottöl | 07 Pineöl |
| 03 Cedernholzöl | 08 Pfefferminzöl |
| 04 Nelkenblätteröl | 09 Patschouliöl |
| 05 Zitronenöl | 010 Lavandinöl. |

In der nachstehenden Tabelle 1 sind die Ergebnisse der einzelnen wäßrigen Lösungen aufgeführt, wobei x = trübe Lösung, xx = schwach getrübte Lösung und xxx = klare, stabile Lösung bedeutet. Ferner ist in der Tabelle vermerkt, wieviel verschiedene Öle ein Lösungsvermittler zu lösen vermag. Je mehr der verwendeten Öle ein Lösungsvermittler zu lösen vermag, desto besser ist er allgemein als Lösungsvermittler zu beurteilen, da er genereller einsetzbar ist. Die in der Tabelle angegebenen Ergebnisse können durch ein höheres Verhältnis von Lösungsvermittler : Parfümöl naturgemäß verbessert werden.

Tabelle 1

| Lösungs-vermittler | Verhältnis LV : Öl | Öl 1 | | | | | | | | | | Zahl der gelösten Öle |
|---|---|---|---|---|---|---|---|---|---|---|---|---|
| | | 01 | 02 | 03 | 04 | 05 | 06 | 07 | 08 | 09 | 010 | |
| A | 7 : 3 | xxx | xx | x | xxx | xx | xxx | xxx | x | x | x | |
| | 8 : 2 | xxx | xxx | xxx | xxx | xxx | xxx | xxx | xxx | xxx | xxx | 10 |
| B | 7 : 3 | xxx | x | x | xx | xx | xxx | xxx | x | x | x | |
| | 8 : 2 | xxx | xxx | xxx | xxx | xxx | xxx | xxx | xxx | xxx | xxx | 10 |
| C | 7 : 3 | xxx | xxx | xx | xxx | xx | xxx | xxx | x | x | xx | |
| | 8 : 2 | xxx | xxx | xxx | xxx | xxx | xxx | xxx | xxx | xxx | xxx | 10 |
| D | 7 : 3 | x | x | x | x | x | x | xxx | x | x | x | |
| | 8 : 2 | xx | xxx | x | xxx | xx | xxx | xxx | xx | xx | xx | 4 |
| E | 7 : 3 | x | x | x | xxx | x | x | x | x | x | xxx | |
| | 8 : 2 | xxx | xxx | xx | xxx | xx | xxx | xxx | xxx | xx | xxx | 7 |
| F | 7 : 3 | xx | xx | x | xx | xx | xx | xxx | x | xx | x | |
| | 8 : 2 | xxx | xxx | xx | xxx | xxx | xxx | xxx | xx | xxx | xx | 7 |
| G | 7 : 3 | xxx | x | x | xx | xx | xx | xx | x | x | x | |
| | 8 : 2 | xxx | xxx | xxx | xxx | xxx | xxx | xxx | xxx | xxx | xxx | 9 |

**Ansprüche**

1. Klare, stabile, wäßrige oder wäßrig-alkoholische Lösungen fettlöslicher Parfümöle, welche als Lösungsvermittler Hydroxyalkyletheroxalkylate enthalten, die aus Epoxyalkanen der Formel

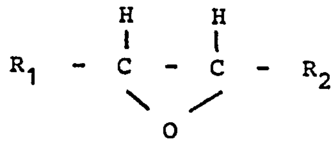

in der $R_1$ und $R_2$ Wasserstoff oder Alkylreste darstellen durch Umsetzung mit einem Alkohol und nachfolgende Oxalkylierung erhältlich sind dadurch gekennzeichnet, daß man als Lösungsvermittler Produkte einsetzt, die durch Umsetzung von Epoxyalkanen der vorgenannten Formel, in welcher $R_1$ und $R_2$ zusammen 6 bis 20 Kohlenstoffatome enthalten, mit polyfunktionellen Alkoholen mit bis zu 10 Kohlenstoffatomen und bis zu 4 Hydroxylgruppen im Molverhältnis Epoxyalkan : Alkohol von 1 : 1,1 bis 1 : 10 bei 50 °C-130 °C in Abwesenheit von Lösungsmitteln in Gegenwart von 0,05 bis 10 g konzentrierter Schwefelsäure oder aromatischen Sulfonsäuren mit höchstens 8 Kohlenstoffatomen pro Mol umzusetzendes Epoxyalkan und nach Neutralisation der Säure und Abdestillieren des nicht umgesetzten Alkohols durch weitere Umsetzung des erhaltenen Reaktionsproduktes mit zunächst 0,5 bis 4 Mol Propylenoxid und anschließend 7 bis 14 Mol Ethylenoxid pro Mol Etheralkohol erhalten werden.

2. Klare, stabile, wäßrige oder wäßrig-alkoholische Lösungen fettlöslicher Parfümöle nach Anspruch 1, dadurch gekennzeichnet, daß man als Lösungsvermittler Produkte einsetzt, die durch Umsetzung der Epoxyalkane mit Ethylenglykol im Molverhältnis Epoxyalkan : Ethylenglykol von 1 : 2 bis 1 : 6 und durch Umsetzung des erhaltenen Reaktionsprodukts mit zunächst 1-1,2 Mol Propylenoxid und anschließend 9-11 Mol Ethylenoxid erhalten wurden.

3. Klare, stabile, wäßrige oder wäßrig-alkoholische Lösungen fettlöslicher Parfümöle nach Anspruch 1 und 2, gekennzeichnet durch einen Gehalt an Hydroxyalkyletheralkoxylaten vorgenannter Zusammensetzung in einer Menge von 0,1 bis 20 Gewichtsprozent, vorzugsweise 0,5 bis 5 Gewichtsprozent, bezogen auf die gesamte Lösung.

**Claims**

1. Clear, stable, aqueous or aqueous-alcoholic solutions of fat-soluble perfume oils containing as solution promoter hydroxyalkyl ether alkoxylates obtainable from epoxyalkanes corresponding to the following formula

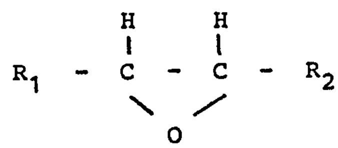

in which $R_1$ and $R_2$ represent hydrogen or alkyl radicals, by reaction with an alcohol, followed by alkoxylation, characterised in that the solution promoters used are products obtained by the reaction of epoxyalkanes corresponding to the above formula, in which $R_1$ and $R_2$ together contain from 6 to 20 carbon atoms, with polyfunctional alcohols containing up to 10 carbon atoms and up to 4 hydroxyl groups in a molar ratio of epoxyalkane to alcohol of from 1 : 1.1 to 1 : 10 at 50-130 °C in the absence of solvents and in the presence of from 0.05 to 10 g of concentrated sulfuric acid or aromatic sulfonic acids containing at most 8 carbon atoms per mole of epoxyalkane to be reacted and, after neutralisation of the acid and removal of the unreacted alcohol by distillation, by further reaction of the reaction product obtained first with 0.5 to 4 moles of propylene oxide and then with 7 to 14 moles of ethylene oxide per mole of ether alcohol.

2. Clear, stable, aqueous or aqueous-alcoholic solutions of fat-soluble perfume oils as claimed in Claim 1, characterised in that the solution promoters used are products obtained by reaction of the epoxyalkanes with ethylene glycol in a molar ratio of epoxyalkane to ethylene glycol of from 1 : 2 to 1 : 6 and by reaction of the reaction product obtained first with 1-1.2 moles of propylene oxide and then with 9 to 11 moles of ethylene oxide.

3. Clear, stable, aqueous or aqueous-alcoholic solutions of fat-soluble perfume oils as claimed in Claims 1 and 2, characterised by a content of hydroxyalkyl ether alkoxylates having the above-mentioned composition in a quantity of from 0.1 to 20 % by weight and preferably in a quantity of from 0.5 to 5 % by weight, based on the solution as a whole.

**Revendications**

1. Solutions aqueuses ou hydroalcooliques stables, claires de parfums solubles dans les huiles, qui contiennent comme adjuvants de dissolution, des oxalkylates d'hydroxyalkyléthers, lesquels peuvent être obtenus à partir d'époxyalcanes de formule

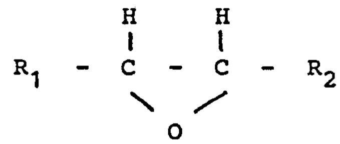

dans laquelle $R_1$ et $R_2$ représentent de l'hydrogène ou des radicaux alkyle, par réaction avec un alcool et oxalkylation subséquente, caractérisées en ce qu'on met en œuvre, comme adjuvants de dissolution, des produits qui sont obtenus par réaction d'époxyalcanes de la formule précédente, dans laquelle $R_1$ et $R_2$ contiennent ensemble 6 à 20 atomes de carbone, avec des alcools polyfonctionnels contenant jusqu'à 10 atomes de carbone et jusqu'à 4 groupes hydroxyle, le rapport molaire époxyalcane : alcool étant de 1 : 1,1 à 1 : 10, à 50 °C-130 °C en l'absence de solvants, en présence de 0,05 à 10 g d'acide sulfurique concentré ou d'acides sulfoniques aromatiques comportant au maximum 8 atomes de carbone par mole d'époxyalcane à transformer et après neutralisation de l'acide et enlèvement par distillation de l'alcool non réagi, par réaction ultérieure du produit de réaction obtenu, d'abord avec 0,5 à 4 moles d'oxyde de propylène et ensuite avec 7 à 14 moles d'oxyde d'éthylène par mole d'étheralcool.

2. Solutions aqueuses ou hydroalcooliques stables, claires de parfums solubles dans les huiles selon la revendication 1, caractérisées en ce qu'on met en œuvre comme adjuvants de dissolution, des produits qui ont été obtenus par réaction d'époxyalcanes avec de l'éthylèneglycol en rapport molaire d'époxyalcane : éthylèneglycol de 1 : 2 à 1 : 6 et par réaction du produit de réaction ainsi obtenu d'abord avec 1-1,2 mole d'oxyde de propylène et ensuite avec 9-11 moles d'oxyde d'éthylène.

3. Solutions aqueuses ou hydroalcooliques stables, claires de parfums solubles dans les huiles selon les revendications 1 et 2, caractérisées en ce qu'elles contiennent les alkoxylates d'hydroxyalkyléther de composition précitée en une quantité de 0,1 à 20 pourcent en poids, de préférence 0,5 à 5 pourcent en poids par rapport à l'ensemble de la solution.